# FASCICULE DE BREVET EUROPEEN

(11) **EP 0 964 000 B1**
(45) Date de publication et mention de la délivrance du brevet: **04.07.2012**
(21) Numéro de dépôt: 99401364.7
(22) Date de dépôt: 07.06.1999
(51) Int. Cl.: C08B 30/14, C08B 30/06, C08L 3/02, A61K 9/20, A61K 9/48, A61K 9/16

(54) **Composition diluante et désintégrante, son procédé d'obtention et son utilisation**
Hilfsmittel- und Sprengmittelzusammensetzung, Verfahren zu deren Herstellung und Verwendung
Excipient and disintegrant composition, process for obtaining the same and its use

(30) Priorité: 08.06.1998 FR 9807175
(43) Date de publication de la demande: 15.12.1999
(73) Titulaire: Roquette Frères, 62136 Lestrem (FR)
(72) Inventeur: Lefevre, Philippe, 59660 Merville (FR); Quettier, Claude, 59253 La Gorgue (FR)
(74) Mandataire: Boulinguiez, Didier

(56) Documents cités:
- EP-A- 0 402 186
- WO-A1-97/37827
- WO-A2-96/22110
- FR-A- 2 661 317
- DATABASE WPI Week 8238 Derwent Publications Ltd., London, GB; AN 82-80106e XP002093553 "Starch adhesive for corrugated cardboard - comprises high amylose corn starch and starch having grain size finer than 40 mesh" & JP 57 131274 A (SUGIYAMA KAGAKY SANGYO), 14 août 1982 (1982-08-14)

## Description

L'invention a pour objet une composition diluante et désintégrante. Elle vise également un procédé d'obtention de cette composition ainsi que l'utilisation de celle-ci dans l'industrie, pour la fabrication de formes solides.

Par l'expression "formes solides", on désigne toute présentation de poudre(s) sous forme de comprimés, pellets, gélules, microsphères ou granulés. Les formes solides consistent essentiellement en matériaux inertes, regroupés sous le terme d'excipients, en complément d'une ou plusieurs substances actives pharmaceutiques, cosmétiques, alimentaires, chimiques ou agrochimiques, telles que arômes, parfums, détergents, pesticides, antibiotiques, enzymes, vitamines. Ces excipients sont généralement classés selon leur(s) fonction(s) principale(s), c'est ainsi que l'on distingue les diluants, ou agents de remplissage, les liants qui assurent la cohésion des ingrédients entre eux, les désintégrants qui permettent la destruction de l'intégrité physique des formes solides lorsque celles-ci sont placées dans un fluide approprié. D'autres excipients peuvent être parallèlement ajoutés, notamment des lubrifiants afin d'améliorer les propriétés d'écoulement des poudres. Un bon diluant de poudre doit posséder les propriétés suivantes :
- compatibilité chimique avec l'actif,
- écoulement libre, pour permettre un remplissage régulier des matrices dans les machines de formage modernes à cadence élevée,
- granulométrie adaptée à celle de l'actif, afin d'assurer un dosage constant,
- absence de poussière, pour faciliter la manipulation, éviter l'encrassement et limiter les risques d'explosion,
- densité élevée, pour favoriser l'écoulement et limiter la taille de la forme solide finale,
- cohésion, pour assurer la stabilité physique des formes solides.

Un bon désintégrant doit quant à lui assurer une disponibilité rapide des substances actives, tout en présentant des propriétés rhéologiques satisfaisantes.

Le formulateur souhaite disposer d'un excipient principal possédant des propriétés optimales, qu'il complétera en fonction des caractéristiques de la forme solide qu'il souhaite développer, avec un ou plusieurs excipients secondaires. Il n'existe en effet pas d'excipient universel présentant l'ensemble des propriétés précédemment décrites.

L'amidon et ses dérivés font partie des excipients de choix, offrant un large spectre de performances pour la formulation de formes solides.

A l'état natif, l'amidon se présente sous forme de granules, dont le diamètre varie approximativement, entre 1 et 100µm.

Sa disponibilité et son faible coût, ainsi que son origine naturelle sont les principaux facteurs favorisant son utilisation. La majorité des amidons du commerce proviennent du maïs, mais le blé et la pomme de terre en sont également des sources importantes. D'autres sources sont connues, comme le riz, le manioc, le pois. L'amidon consiste en deux types de polymères d'α-D-glucose, l'amylose et l'amylopectine, qui ont une structure différente : l'amylose est un polymère linéaire, tandis que l'amylopectine possède une structure ramifiée.

La plupart des amidons, provenant du maïs, du blé ou de la pomme de terre, contiennent 18 à 28% d'amylose.

Dans la fabrication de formes solides, l'amidon est utilisé en tant que diluant, liant ou désintégrant.

L'amidon natif s'offre, à lui seul, à un nombre limité d'applications. En effet, lorsqu'il est utilisé comme diluant de compression directe, sa comprimabilité est insuffisante pour permettre la fabrication de comprimés de dureté satisfaisante.

En effet, dans le cas particulier de la compression, les particules subissent une déformation, qui peut être de différents types suivant leur nature : déformation élastique ou plastique.
- lors d'une déformation élastique, la déformation disparaît lorsque la force cesse d'être appliquée. Cette déformation n'est pas favorable à l'obtention de comprimés, les particules retrouvant leur état initial en fin de compression. Ceci est notamment le cas des amidons natifs.
- lors d'une déformation plastique, au contraire, la déformation persiste lorsque la force cesse d'être appliquée, ceci étant tout à fait favorable à l'obtention de comprimés.

De plus, l'amidon natif possède de mauvaises propriétés d'écoulement, ce qui fait qu'on le déconseille dans les formulations pour compression directe. Ceci est dû à la faible taille de ses particules, ainsi qu'à sa faible densité. Or, la fluidité des formulations est un critère essentiel qui conditionne l'uniformité de poids des comprimés terminés. Par contre, l'amidon natif possède de bonnes propriétés désintégrantes. En effet, les granules d'amidon gonflent en présence d'eau, ce qui provoque l'éclatement de la structure dans laquelle ils sont contenus, et donc sa désintégration.

L'amidon natif peut être modifié de façon simple et peu coûteuse par un traitement thermique provoquant l'éclatement des granules et une hydrolyse partielle des chaînes polymériques. On obtient alors un amidon prégélatinisé qui, sous forme de poudre et pour une granulométrie sélectionnée, est un produit s'écoulant et se comprimant bien, mais possédant un pouvoir désintégrant pratiquement nul. Il est préférentiellement utilisé comme liant de poudres.

Des modifications chimiques, telles que la réticulation, décrite dans le brevet US 4 369 308, peuvent être apportées à l'amidon prégélatinisé, mais celles-ci conduisent à un agent désintégrant de mauvaise qualité.

Compte-tenu du fait que les propriétés de l'amidon prégélatinisé et de l'amidon natif sont diamétralement opposées mais néanmoins conjointement requises dans de nombreuses formes solides, un mélange simple des deux poudres aurait pu être envisagé. Cependant, ces deux amidons possèdent des granulométries très éloignées l'une de l'autre, ce qui entraîne un "démélange" rapide des poudres dès la moindre manipulation, et une homogénéité de la forme solide finale très fluctuante, ce qui est industriellement inacceptable.

Il a donc été proposé, dans le brevet FR 1 583 232, un procédé de compactage conduisant à un amidon partiellement prégélatinisé. Le produit obtenu selon ce procédé, et commercialisé sous le nom de STARCH 1500^{®}, possède de bonnes propriétés liantes et diluantes, mais son pouvoir désintégrant reste limité, et ses propriétés d'écoulement sont médiocres, ce qui nécessite l'emploi par les formulateurs d'excipients complémentaires (VISAVARUNGROJ N, RENON J.P., (1992) Pharm. Tech. Int. JAN/FEB p26-32).

De plus, la mise en oeuvre de ce procédé, complexe et coûteuse, ne permet pas une bonne maîtrise de la granulométrie, ce qui entraîne une forte variation des propriétés qu'offre ce produit.

Plus récemment, un autre procédé d'obtention d'amidon prégélatinisé directement comprimable a été proposé, dans le brevet EP 402 186. Le produit obtenu, commercialisé sous le nom de SEPISTAB^{®} ST 200, résulte d'une granulation humide d'amidon natif par une solution d'empois d'amidon. Ce procédé, qui nécessite une forte proportion d'amidon natif, conduit à une poudre dont les granules sont friables et fragiles, ce qui nuit aux utilisations de cette poudre sur machines industrielles à cadences élevées, qui engendrent un stress physique important.

La demande WO 96/22110 divulgue une technique de stérilisation de l'amidon à sec par inhibition thermique. Ce procédé consiste en une première étape de déshydratation de l'amidon et une seconde étape de chauffage. L'amidon décrit dans cette demande a, en fonction de la nature de l'amidon utilisé, des propriétés différentes. Ainsi, l'amidon décrit dans la demande WO 96/22110 peut avoir des propriétés diluantes ou des propriétés désintégrantes, mais il n'est fait aucunement mention d'un amidon ayant ces deux propriétés à la fois.

Par conséquent, aucun des procédés de l'art antérieur ne permet d'obtenir, de façon économiquement acceptable, des compositions d'amidon présentant simultanément de bonnes propriétés diluantes et désintégrantes.

L'invention a donc pour but de remédier aux inconvénients de l'art antérieur et de fournir une composition d'amidon diluante et désintégrante telle que définie à la revendication 1 répondant mieux que celles qui existent déjà aux diverses exigences de la pratique.

La Société Demanderesse a eu le mérite de trouver que ce but était atteint dès lors que l'on utilise un amidon prégélatinisé contenant une teneur non négligeable de granules intacts d'amidon riche en amylose.

De manière plus précise, la composition diluante et désintégrante conforme à l'invention est caractérisée par le fait qu'elle contient une proportion efficace de grains intacts d'amidon riche en amylose inclus dans une matrice d'amidon prégélatinisé.

L'invention se rapporte également à un procédé de préparation d'une composition diluante et désintégrante telle que définie à la revendication 1.

On désigne par proportion efficace celle nécessaire et suffisante pour obtenir l'effet recherché, c'est-à-dire de bonnes propriétés diluantes et désintégrantes.

Par le terme "amidon prégélatinisé", on désigne tout amidon ayant subi un traitement thermique en présence d'eau, de sorte qu'il perde sa structure granulaire, et devienne plus ou moins soluble dans l'eau froide. On désigne par "amidon" les amidons de toutes origines, naturels ou hybrides, modifiés ou non, et leurs mélanges quelconques.

Par le terme "amidon riche en amylose", on désigne tout amidon présentant une teneur en amylose au moins égale à 50%, comme notamment les amidons de maïs commercialisés par la Société Demanderesse sous la marque EURYLON^{®}.

La composition diluante et désintégrante conforme à l'invention est constituée de granules intacts d'amidon riche en amylose, c'est-à-dire non éclatés, qui se trouvent enrobés totalement ou partiellement d'amidon prégélatinisé, ce dernier ayant perdu quant à lui, sa structure granulaire. La nature particulière de cette composition lui confère à elle seule l'ensemble des propriétés requises dans la formulation de formes solides, propriétés qui n'avaient jusqu'alors jamais été réunies dans un même produit de l'art antérieur. Ceci permet d'envisager sous un jour nouveau les applications nombreuses de la composition conforme à l'invention, notamment dans la fabrication de comprimés, de gélules, de granulés ou de toute autre forme solide susceptible de nécessiter un excipient possédant lesdites propriétés. D'autre part, l'amidon riche en amylose présente une résistance thermique et mécanique supérieure aux amidons standards, ce qui permet d'envisager des traitements de stérilisation, de granulation-séchage, ou tout autre traitement opéré sur la forme solide pouvant représenter un stress physique.

La composition diluante et désintégrante selon l'invention contient une proportion d'amidon riche en amylose comprise entre 20 et 90%, ce pourcentage étant calculé en poids par rapport au poids total d'amidon riche en amylose et d'amidon prégélatinisé contenu dans ladite composition. De préférence, cette proportion est comprise entre 30 et 80%.

La Demanderesse a en effet mis en évidence, après de longues recherches, que cette seconde caractéristique permettait d'obtenir avantageusement à la fois de bonnes propriétés diluantes et désintégrantes.

Ainsi, au delà de 90% d'amidon riche en amylose dans la composition, il devient généralement impossible de former une matrice d'amidon prégélatinisé incluant des granules d'amylose, et par là même, d'obtenir l'ensemble des propriétés caractérisant la composition selon l'invention. D'autre part, en deçà de 20% d'amidon riche en amylose, les propriétés désintégrantes ne sont généralement pas satisfaisantes.

En ce qui concerne la densité apparente de la composition selon l'invention, qui représente un critère d'intérêt pour le formulateur, celle-ci est avantageusement supérieure à 0,5 g/ml.

La densité apparente ou masse volumique apparente avant tassement a été mesurée selon la méthode analytique 2.9.15 de la Pharmacopée Européenne, 3ème édition.

Le principe de cette mesure repose sur la détermination des volumes avant et après tassement des poudres et met en oeuvre un appareillage constitué d'un appareil de tassement muni d'une éprouvette graduée, le dit appareil provoquant des chutes successives de l'éprouvette contenant la poudre à tester.

La Demanderesse a ainsi mis en évidence qu'une densité apparente au moins égale à 0,5 g/ml permettait d'obtenir un écoulement particulièrement satisfaisant de la composition selon l'invention, et que pour son utilisation notamment dans le remplissage de gélules, une densité élevée permettait de réduire la taille de la gélule, ce qui est tout à fait indiqué pour faciliter son ingestion par le patient dans le cas de gélules pharmaceutiques. Une composition diluante et désintégrante contenant au moins 10% en poids d'amidon totalement prégélatinisé, ce pourcentage étant exprimé par rapport au poids total d'amidon contenu dans ladite composition, et présentant une densité apparente supérieure à 0,5 g/ml, constitue un produit nouveau, qui se distingue des amidons prégélatinisés de l'art antérieur.

La Demanderesse a par ailleurs mis en évidence que la granulométrie de la composition selon l'invention pouvait se situer dans une gamme très large, sans que les propriétés diluantes et désintégrantes n'en soient affectées. Cette caractéristique a été calculée à partir des refus obtenus par tamisage sur tamis successifs d'ouvertures décroissantes. La granulométrie moyenne de la composition selon l'invention peut notamment être comprise entre 50µm et 1000µm.

Ceci permet avantageusement d'adapter à loisir la granulométrie de la composition selon l'invention à celle de la substance active, tout en conservant l'ensemble des propriétés précédemment citées.

Les compositions diluantes et désintégrantes conformes à l'invention sont susceptibles d'être obtenues selon une multitude de variantes mais tout particulièrement selon un procédé comportant les étapes suivantes :
- préparation d'un lait d'amidons contenant de 20 à 90 % en poids d'amidon dont la teneur en amylose est au moins égale à 50 %, ces pourcentages étant exprimés par rapport au poids total d'amidon et d'amidon riche en amylose contenus dans le lait,
- cuisson de ce lait à une température inférieure à 130°C et de préférence inférieure à 110°C de façon à obtenir une pâte,
- séchage de cette pâte,
- broyage de la pâte séchée,
- récupération de la composition d'amidon diluante et désintégrante ainsi obtenue.

Les caractéristiques peuvent être ajustées en modifiant la proportion d'amidon riche en amylose du lait de départ.

En ce qui concerne la préparation du lait, on préfère que celui-ci ait une matière sèche d'au moins 30%.

Les étapes de cuisson et de séchage peuvent être mises en oeuvre par toute technique connue de l'homme de métier.

En ce qui concerne la température de cuisson du lait, on préfère qu'elle soit voisine de 100°C.

De même, le broyage est conduit selon tout type de technique connue permettant d'obtenir une poudre possédant les caractéristiques granulométriques recherchées.

Selon un mode de réalisation avantageux du procédé conforme à l'invention, on prépare un lait contenant de 20 de 30 à 80% d'amidon riche en amylose, ce pourcentage étant exprimé en poids d'amidon riche en amylose par rapport au poids total d'amidons contenus dans le lait.

Selon un mode de réalisation avantageux du susdit procédé, la température de cuisson du lait d'amidons est comprise entre 80 et 105°C.

La Société Demanderesse a démontré que l'on pouvait avantageusement fabriquer la composition conforme à l'invention en utilisant un tambour-sécheur. Un tel équipement permet de reproduire sur un seul et même dispositif les étapes de cuisson et de séchage du procédé conforme à l'invention.

Ce matériel bien connu permet, en effet, en exploitant la chaleur transférée depuis la surface des tambours chauffés à la vapeur jusqu'au lait d'amidons, de gélatiniser ce lait, qui est étalé uniformément en une mince pellicule sur la surface chaude du tambour par des éléments applicateurs.

Le film ainsi formé est ensuite raclé à l'aide d'un couteau racleur, de manière à décoller une feuille qui est ensuite broyée de façon à obtenir une composition conforme à l'invention.

Un avantage important du procédé conforme à l'invention réside dans le fait que sa mise en oeuvre appliquée au lait d'amidons conforme à l'invention est simple et peu coûteuse. Il permet d'autre part d'obtenir une composition homogène, consistant en grains intacts d'amidon riche en amylose inclus dans une matrice d'amidon prégélatinisé, qui ne risque pas de provoquer une ségrégation lors de sa mise en oeuvre pour la fabrication de formes solides. En outre, le procédé permet de décliner une large gamme de compositions désintégrantes conformes à l'invention, en ajustant les proportions, au sein du mélange, de l'amidon et de l'amidon riche en amylose, sans que ce soit au détriment des propriétés diluantes desdites compositions. De plus, il est possible, à tout moment de ce procédé, y compris donc avant, pendant et/ou après l'étape de broyage sus décrite, de mettre la composition amylacée en présence d'un ou plusieurs constituants non amylacés tels que, par exemple, des substances actives, des conservateurs, des excipients, ayant ou non des propriétés diluantes ou désintégrantes, dès lors que de tels constituants ne nuisent pas aux propriétés recherchées du mélange final.

En tout état de causé, les compositions conformes à l'invention possèdent des propriétés diluantes et désintégrantes supérieures à celles des produits amylacés de l'art antérieur.

Une explication possible de ces propriétés remarquables est que le traitement de cuisson-séchage appliqué au lait d'amidons, provoque une densification inattendue du mélange, tout en préservant des granules d'amidon riche en amylose intacts en proportion efficace qui assurent, quant à eux, le pouvoir désintégrant recherché, et une résistance physique accrue.

La composition conforme à l'invention peut ainsi être avantageusement utilisée dans la fabrication de formes solides, en tant qu'agent diluant et désintégrant, que ce soit dans les domaines alimentaires, pharmaceutiques, cosmétiques, chimiques ou agrochimiques.

Les avantages de la présente invention seront mieux compris à la lecture des exemples suivants et des figures qui s'y rapportent, donnés à titre purement illustratif.

### Exemple 1

### Préparation de compositions selon l'invention et comparaison avec des compositions de l'art antérieur.

On prépare deux compositions contenant respectivement 50 et 75 % d'amidon riche en amylose dans les conditions suivantes :
Composition 1A :
   50% d'amidon de maïs standard
   50% d'EURYLON^{®} 7
Composition 1B :
   25% d'amidon de maïs standard
   75% d'EURYLON^{®} 7
Matière sèche des laits : 35%

On cuit chaque lait ainsi préparé sur tambour-sécheur monocylindre, à une température de 100°C.

Les principales caractéristiques physiques des compositions 1A et 1B ainsi obtenues sont données dans le tableau suivant, en comparaison avec un amidon de maïs standard et un amidon de maïs prégélatinisé commercialisé par la Demanderesse sous la marque LYCATAB PGS^{®}

| | **1A** | **1B** | **Amidon de maïs** | **LYCATAB PGS** |
|---|---|---|---|---|
| **Diamètre moyen (1)** µ**m (microns)** | 85 | 90 | 13,8-14,5 | 90 |
| **Masse volumique apparente avant tassement (2) g/ml** | 0,65 | 0,64 | 0,43 | 0,45 |
| **Aptitude à l'écoulement (3) (secondes)** | 5 | 6 | ind. | 9 |
| **Teneur en amidon riche en amylose (%/sec)** | 50 | 75 | 0 | 0 |
| **Teneur en amidon standard (%/sec)** | 50 | 25 | 100 | 100 |

| | | | | |
|---|---|---|---|---|
| (1) Le diamètre moyen est calculé à partir de la granulométrie mesurée par tamisage sur tamis successifs de 200, 100, 80, 63 et 40 µm (microns), sauf pour l'amidon de maïs dont la valeur prise est celle citée dans WHISTLER R.L., BE MILLER J.N., PASCHALL E.F., (1984), Starch Chem. and Techn., 2nd ed.. (2) La masse volumique apparente est mesurée selon la méthode de pharmacotechnie 2.9.15 de la Pharmacopée Européenne, 3ème édition. (3) L'aptitude à l'écoulement est mesurée selon la méthode de pharmacotechnie 2.9.16 de la Pharmacopée Européenne 3ème édition. | | | | |

On conclut de ces résultats que les compositions selon l'invention possèdent une densité apparente supérieure aux produits de l'art antérieur, et une meilleure aptitude à l'écoulement.

### Figures 1 à 7

### Observation en microscopie électronique des compositions 1A et 1B selon l'exemple 1.

Les compositions observées présentent un aspect très différent des amidons prégélatinisés ordinaires : elles se présentent en effet sous formes arrondies, de type granulés. Les granules d'amidon riche en amylose sont particulièrement visibles dans la composition lB, ils sont nettement visibles dans les cavités. Ces granules sont recouverts d'amidon déstructuré.

### Exemple 2

### Evaluation du pouvoir désintégrant des compositions préparées selon l'exemple 1, Comparaison avec des compositions de l'art antérieur.

On évalue les propriétés désintégrantes selon le test suivant :

Sur une presse alternative de type FROGERAIS AM sont réalisés des comprimés plats, de diamètre 13mm, d'épaisseur 5mm, de poids 1g et de composition suivante :
- compositions 1A ou 1B selon l'exemple 1.
- phosphate dicalcique dihydraté pour compression directe (EMCOMPRESS^{®}) : 50%
- stéarate de magnésium : 0,5%
(EMCOMPRESS^{®} est commercialisé par la société MENDELL)

Les temps de désagrégation de ces comprimés sont mesurés selon la méthode de pharmacotechnie 2.9.1 de la Pharmacopée Européenne, 3ème édition.

Les temps de désagrégation indiqués ci-dessous sont les temps nécessaires à la désagrégation totale du comprimé.

| **Produits** | **1A** | **1B** | **Amidon prégélatinisé** | **STARCH 1500** |
|---|---|---|---|---|
| **Temps de désagrégation** | 2mn 50s | 1mn 45s | > 15 mn | 2mn 45 s |

On conclut de ces résultats que les compositions conformes à l'invention possèdent une fonction désintégrante nette, équivalente aux compositions de l'art antérieur lorsque la teneur en EURYLON^{®} est de 50%, supérieure pour une teneur de 75% en EURYLON^{®}.

Les granules d'amylose inclus dans la matrice d'amidon prégélatinisé ont donc une fonction désintégrante suffisante pour s'opposer à l'action liante de l'amidon prégélatinisé.

### Exemple 3

### Evaluation des compositions conformes à l'invention dans le remplissage de gélules. Comparaison avec des compositions de l'art antérieur.

On fabrique une carotte pour le remplissage de gélules de la manière suivante : on utilise une matrice métallique traversée d'un trou vertical de forme cylindrique et de diamètre 6,3 mm et de hauteur de 50 mm.

Dans un premier temps, l'extrémité inférieure du trou est fermée par une plaque métallique. Puis on verse 0,78 ml des compositions d'amidon décrites dans l'exemple 1 ou des produits de l'art antérieur, qui sont tous préalablement adjuvantés de 0,5% de stéarate de magnésium.

Dans un second temps, on exerce par l'intermédiaire d'un poinçon une force sur la poudre jusqu'à l'obtention d'une carotte suffisamment cohésive pour être manipulée.

Dans un troisième temps, on enlève la plaque métallique qui obstrue l'extrémité inférieure du trou et on éjecte la carotte dans une gélule qui est ensuite refermée.

Les mesures effectuées sont le poids initial de poudre correspondant au 0,78 ml de remplissage, la densité finale de la carotte, et le temps de désagrégation de la gélule contenant la carotte. Ce temps de désagrégation est mesuré selon la méthode de pharmacotechnie 2-9-1 de la Pharmacopée Européenne. Le temps indiqué est celui nécessaire à une dissolution totale de l'enveloppe de la gélule et de désagrégation totale de la carotte.

| | **1A** | **1B** | **Amidon de maïs prégélatinisé LYCATAB PGS** | **STARCH 1500** |
|---|---|---|---|---|
| **Poids initial de poudre (en g)** | 0,54 | 0,53 | 0,37 | 0,48 |
| **Densité finale de la carotte** | 1,02 | 0,99 | 0,86 | 0,89 |
| **Temps de désagrégation de la gélule complète** | 5mn 30s | 3mn 20s | > 60 mn | 10 mn |

Les valeurs dé poids initial de poudre et de densité finale de la carotte pour les compositions selon l'invention sont supérieures à celles des compositions de l'art antérieur.

Ces caractéristiques sont particulièrement intéressantes puisqu'elles permettent pour un même poids de contenu et donc de même dosage d'actif de diminuer la taille des gélules, qu'elles soient produites par arasement (en rapport avec le poids initial de poudre) ou sur machines équipées de disque doseur ou compresso-doseur (en rapport avec la densité finale de la carotte).

Or une taille de gélule faible est bénéfique au traitement car elle facilite l'observance par le patient.

Il est de plus remarquable que cette augmentation de densité ne se réalise pas au détriment de la désagrégation : les compositions selon l'invention donnent des désagrégations nettement plus rapides que les compositions de l'art antérieur.

### Exemple 4

### Evaluation de la comprimabilité d'une composition selon l'invention.

On prépare des comprimés à partir d'une composition conforme à l'invention, contenant 50% d'EURYLON^{®} à laquelle on ajoute 0,5% en poids de stéarate de magnésium en tant que lubrifiant.

La compression est effectuée grâce à une presse alternative FROGERAIS de type AM. Cette presse est équipée de poinçons ronds à faces concaves, d'un diamètre égal à 13mm.

On règle sur la presse l'enfoncement du poinçon supérieur et le volume de remplissage de la matrice, de façon à obtenir des comprimés d'épaisseur 5 mm et de densité 1,14.

La dureté des comprimés est mesurée en utilisant un duromètre SCHLEUNIGER-2E.

La composition conforme à l'invention, contenant 50 % d'EURYLON^{®} présente une comprimabilité tout à fait satisfaisante. Celle-ci se traduit par une dureté de 105N.

Il est de plus remarquable qu'une telle dureté ait été obtenue avec du stéarate de magnésium réputé inadapté pour la lubrification et la compression de dérivés amylacés.

### Exemple 5

### Evaluation du pouvoir liant en granulation humide des compositions conforme à l'invention.

La composition selon l'exemple 1A est utilisé comme liant de granulation humide dans la formule suivante :

| | |
|---|---|
| - Paracétamol cristallisé fin | |
| (RHODAPAP/RHONE POULENC) | 250,0g |
| - Amidon de maïs standard (ROQUETTE) | 19,5g |
| - Composition de l'exemple 1A | 36,8g |
| - Eau | 70,0g |

Des granulés sont préparés de la manière suivante : les trois poudres sont mélangées dans un mélangeur planétaire à vitesse minimum pendant 5 minutes, l'eau est pulvérisée lentement sur les poudres en mouvement. Puis l'ensemble est transféré dans un mélangeur à haut cisaillement et mélangé à très grande vitesse pendant 5 minutes.

Enfin, les granulés obtenus sont séchés 30 minutes à 30°C dans un séchoir à lit d'air fluidisé (AEROMATIC).

Le mélange initial de poudre présente moins de 15% de particules de taille supérieure à 200 microns.

Les granulés obtenus ont la granulométrie suivante :

| | |
|---|---|
| - supérieur à 200µm : | 95.5% |
| - supérieur à 315µm : | 84.1% |
| - supérieur à 500µm : | 35.0% |

La comparaison de la granulométrie initiale du mélange des poudres et de la granulométrie des granulés montre que les compositions selon l'invention ont un réel pouvoir liant de granulation humide.

Elles peuvent donc être avantageusement utilisées pour la production de granulés qui pourront être ultérieurement transformés en comprimés après calibrage, lubrification et compression sur presse à comprimer.

### Exemple 6

### Evaluation de la friabilité des compositions conformes à l'invention. Comparaison à des compositions de l'art antérieur.

La friabilité est mesurée en utilisant le test suivant :
- 15 g de poudre sont placés dans le tambour d'un friabilimètre ERWEKA TA (équipé du tambour à ailettes (12) de diamètre 19.5cm). Cinq billes d'acier de diamètre 16,7 mm et de poids total 94,3 g sont ajoutées. L'ensemble est mis en rotation pendant 30 minutes.

Les pourcentages de particules inférieures à 100 microns sont mesurés avant et après ce test.

L'augmentation de ce pourcentage après ce test donne la friabilité de la poudre.

| **Particules inférieures à 100** µ**m** | **1A** | **SEPISTAB ST 200** |
|---|---|---|
| **Pourcentage avant le test** | 58,8 | 31,2 |
| **Pourcentage après le test** | 58,9 | 40,3 |
| **Augmentation (friabilité)** | 0 % | 29,20 % |

Contrairement au produit de l'art antérieur, les compositions selon l'invention ne sont pas friables, ce qui est un avantage indéniable. L'utilisateur pourra faire subir à la poudre un ou plusieurs traitements mécaniquement stressants, si nécessaire pendant une durée très longue sans craindre l'apparition des poussières qui sont à éviter autant lors des procédés de compression que de remplissage de gélules. De plus, l'absence de friabilité est un gage de préservation des propriétés essentielles de la poudre : granulométrie, écoulement, masse volumique avant et après tassement, et ce quels que soient les traitements subis.

Les compositions conformes à l'invention allient donc avantageusement des propriétés qui n'avaient, jusqu'à présent, jamais été trouvées simultanément au sein d'une même composition amylacée. Elles possèdent en effet à la fois des propriétés diluantes et désintégrantes, soit, en d'autres termes, une densité élevée, de bonne propriétés d'écoulement, une granulométrie adaptée, un délitement rapide, et une friabilité pratiquement nulle.

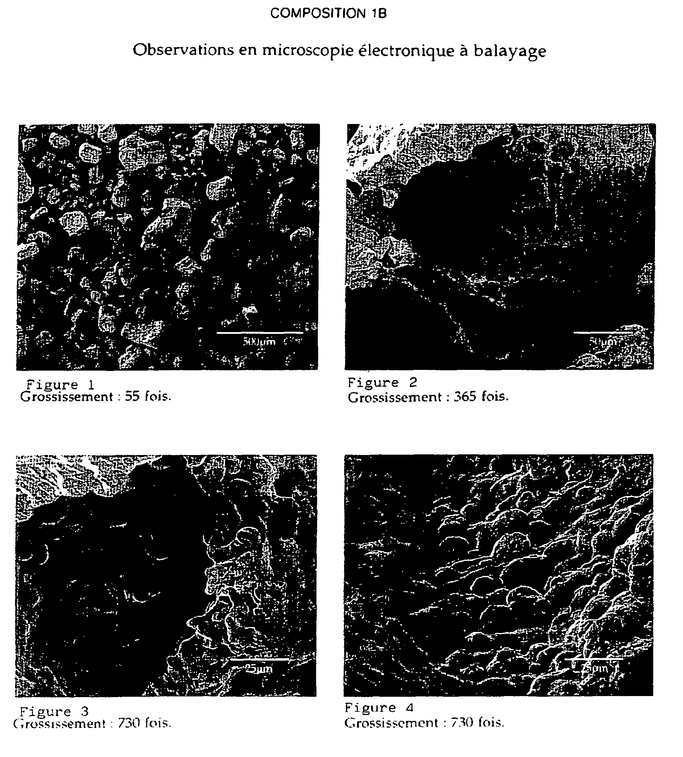

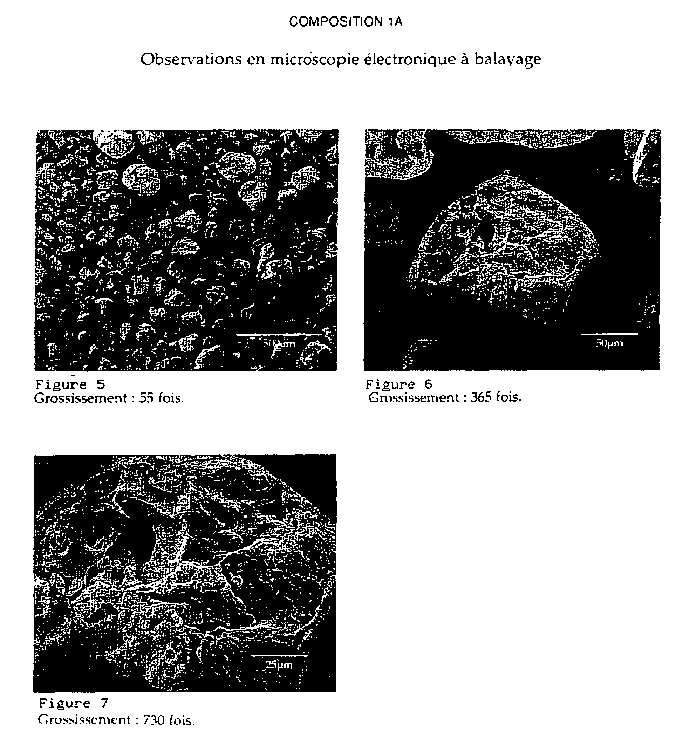

## Revendications

1. Composition diluante et désintégrante, **caractérisée en ce qu'**elle est constituée en proportion efficace de granules intacts d'amidon ayant une teneur en amylose au moins égale à 50%, lesdits granules étant totalement ou partiellement enrobés d'amidon prégélatinisé, ce dernier ayant perdu quant à lui, sa structure granulaire, et **en ce qu'**elle comprend de 20 à 90 % en poids d'amidon ayant une teneur en amylose au moins égale à 50%, ces pourcentages étant exprimés par rapport au poids total d'amidon contenu dans ladite composition.

2. Composition selon la revendication 1, **caractérisée en ce qu'**elle comprend de 30 à 80 % en poids d'amidon ayant une teneur en amylose au moins égale à 50%, ces pourcentages étant exprimés par rapport au poids total d'amidon contenu dans ladite composition.

3. Composition selon la revendication 1 ou 2, **caractérisée en ce qu'**elle présente une densité apparente supérieure à 0,5 g/ml, et/ou une granulométrie moyenne comprise entre 50 et 1000 µm.

4. Composition selon l'une quelconque des revendications 1 à 3, **caractérisée en ce qu'**elle contient au moins 10% en poids d'amidon totalement prégélatinisé ayant perdu sa structure granulaire, ce pourcentage étant exprimé par rapport au poids total d'amidon contenu dans ladite composition, et **en ce qu'**elle présente une densité apparente supérieure à 0,5 g/ml.

5. Procédé d'obtention d'une composition selon l'une quelconque des revendications 1 à 4, **caractérisé en ce qu'**il comprend les étapes consistant à :
- préparer un lait d'amidons contenant de 20 à 90% en poids d'un amidon dont la teneur en amylose est au moins égale à 50%, ces pourcentages étant exprimés par rapport au poids total d'amidon et d'amidon riche en amylose contenus dans le lait,
- cuire le lait ainsi obtenu à une température inférieure à 130 °C , de façon à obtenir une pâte,
- sécher ladite pâte,
- broyer la pâte séchée,
- recueillir la composition diluante et désintégrante ainsi obtenue.

6. Procédé selon la revendication 5, **caractérisé en ce que** le lait contient de 30 à 80% en poids d'amidon riche en amylose, ces pourcentages étant exprimés par rapport au poids total d'amidon contenu et d'amidon riche en amylose contenus dans le lait.

7. Procédé selon l'une ou l'autre des revendications 5 et 6, **caractérisé en ce que** la température de cuisson du lait est comprise entre 80 °C et 105°C.

8. Procédé selon l'une quelconque des revendications 5 à 7, **caractérisé en ce que** la cuisson et le séchage du lait d'amidons sont réalisés sur tambour-sécheur.

9. Procédé selon l'une quelconque des revendications 5 à 8, **caractérisé en ce que** l'on broie la pâte séchée de manière à obtenir une granulométrie comprise entre 50 et 1000 µm.

10. Forme solide **caractérisée en ce qu'**elle comprend une composition conforme à l'une quelconque des revendications 1 à 4, ou obtenue selon l'une quelconque des revendications 5 à 9.

11. Procédé selon la revendication 5, **caractérisé en ce que** la température de cuisson du lait est inférieure à 110 °C.

## Claims

1. A diluent and disintegrating composition, **characterised in that** it consists of an effective proportion of intact starch grains having an amylose content at least equal to 50%, said starch granules being totally or partially coated with pre-gelatinised starch, this latter having lost its granular structure, and **in that** it comprises enclosed in a pre-gelatinised starch matrix, from 20 to 90% by weight of starch having an amylose content of at least 50 %, these percentages being expressed relative to the total starch weight contained in the aforesaid composition.

2. The composition according to claim 1, comprising from 30 to 80% by weight of starch having an amylose content at least equal to 50 %. These percentages being expressed relative to the total starch weight contained in the aforesaid composition.

3. The composition according to claim 1 or 2, having an apparent density greater than 0.5 g/ml, and/or a mean granulometry between 50 and 1000µm.

4. The composition according to anyone of claims 1-3, **characterized in that** it contains at least 10% by weight of totally pre-gelatinised starch, this percentage being expressed relative to the total starch weight contained in the aforesaid composition, and having an apparent density greater than 0.5 g/ml.

5. A process for obtaining a composition according to anyone of claims 1 to 4, comprising:
- preparing a milk of starch containing from 20 to 90 % by weight of starch having an amylose content at least equal to 50 % these percentages being expressed relative to the total weight of the starch and amylose rich starch contained in the milk,
- cooking the milk thus produced at a temperature less than 130°C, so as to obtain a paste,
- drying aforesaid paste,
- grinding the dried paste,
- collecting the diluent and disintegrating composition thus produced.

6. The process according to claim 5, wherein the milk contains from 30 to 80 % by weight of amylose rich starch, these percentages being expressed relative to the total weight of the starch and amylose rich starch contained in the milk.

7. The process according to claim 5 or 6, wherein the cooking temperature of the milk is comprised between 80 and 105 °C.

8. The process according to anyone of claims 5 to 7, wherein the cooking and drying of the starch milk is carried out on a drum drier.

9. The process according to anyone of claims 5 to 8, wherein the dried paste is ground so as to obtain a granulometry between 50 and 1000µm.

10. A solid shape comprising a composition according to anyone of claims 1 to 4, or produced according to anyone of claims 5 to 9.

11. The process according to claim 5, **characterized in that** the cooking temperature of the milk is less than 110 °C.

## Patentansprüche

1. Verdünnungs- und Sprengmittelzusammensetzung, **dadurch gekennzeichnet, dass** sie aus einem wirkungsvollen Anteil aus intakten Stärkekörnern besteht, mit einem Amylosegehalt von mindestens gleich 50 %, wobei die Körnern vollständig oder teilweise von vorgelatinierter Stärke umhüllt sind, wobei die letztere ihrerseits ihre granuläre Struktur verloren hat, und **dadurch**, dass sie 20 bis 90 Gew.-% Stärke mit einem Amylosegehalt von mindestens gleich 50 % umfasst, wobei diese Prozentanteile bezüglich des Gesamtgewichts von Stärke, die in der Zusammensetzung enthalten ist, angegeben sind.

2. Zusammensetzung nach Anspruch 1, **dadurch gekennzeichnet, dass** sie von 30 bis 80 Gew.-% Stärke mit einem Amylosegehalt von mindestens gleich 50 % umfasst, wobei diese Prozentanteile bezüglich des Gesamtgewichts von Stärke, die in der Zusammensetzung enthalten ist, angegeben sind.

3. Zusammensetzung nach Anspruch 1 oder 2, **dadurch gekennzeichnet, dass** sie eine scheinbare Dichte von über 0,5 g/ml aufweist und/oder eine mittlere Körnung zwischen 50 und 1000 µm.

4. Zusammensetzung nach Anspruch 1 bis 3, **dadurch gekennzeichnet, dass** sie mindestens 10 Gew.-% vollständig vorgelatinierte Stärke, die ihre granuläre Struktur verloren hat, enthält, wobei dieser Prozentanteil bezüglich des Gesamtgewicht der in der Zusammensetzung enthaltenen Stärke angegeben ist, und **dadurch**, dass sie eine scheinbare Dichte von über 0,5 g/l aufweist.

5. Verfahren zum Erhalten einer Zusammensetzung nach einem der Ansprüche 1 bis 4, **dadurch gekennzeichnet, dass** es die folgenden Schritte umfasst:
- Herstellen einer Stärkemilch, enthaltend von 20 bis 90 Gew.-% einer Stärke, deren Amylosegehalt mindestens gleich 50 % ist, wobei die Prozentanteile bezüglich des Gesamtgewichts von Stärke und Amylosereicher Stärke, die in der Milch enthalten sind, angegeben sind,
- Kochen der so erhaltenen Milch bei einer Temperatur unter 130 °C, um einen Brei zu erhalten,
- Trocknen des Breis,
- Zerkleinern des getrockneten Breis,
- Sammeln der so erhaltenen Verdünnungs- und Sprengmittelzusammensetzung.

6. Verfahren nach Anspruch 5, **dadurch gekennzeichnet, dass** die Milch 30 bis 80 Gew.-% Amylose-reiche Stärke enthält, wobei die Prozentanteile bezüglich des Gesamtgewichts von Stärke und Amylosereicher Stärke, die in der Milch enthalten sind, angegeben sind.

7. Verfahren nach einem der Ansprüche 5 oder 6, **dadurch gekennzeichnet, dass** die Kochtemperatur der Milch zwischen 80 °C und 105 °C Liegt.

8. Verfahren nach einem der Ansprüche 5 bis 7, **dadurch gekennzeichnet, dass** das Kochen und das Trocknen der Stärkemilch auf einem Trommeltrockner erfolgen.

9. Verfahren nach einem der Ansprüche 5 bis 8, **dadurch gekennzeichnet, dass** man den getrockneten Brei so zerkleinert, um eine Körnung zwischen 50 und 1000 µm zu erhalten.

10. Feste Form, **dadurch gekennzeichnet, dass** sie eine Zusammensetzung nach einem der Ansprüche 1 bis 4 oder eine Zusammensetzung, erhalten nach einem der Ansprüche 5 bis 9, umfasst.

11. Verfahren nach Anspruch 5, **dadurch gekennzeichnet, dass** die Kochtemperatur der Milch unter 110 °C liegt.
